# EUROPEAN PATENT SPECIFICATION

(11) **EP 3 560 469 B1**
(45) Date of publication and mention of the grant of the patent: **16.06.2021**
(21) Application number: 18168885.4
(22) Date of filing: 24.04.2018
(51) Int. Cl.: A61F 13/533, A61F 13/539, A61F 13/15

(54) **ABSORBENT ARTICLE**
SAUGFÄHIGER ARTIKEL
ARTICLE ABSORBANT

(43) Date of publication of application: 30.10.2019
(73) Proprietor: Drylock Technologies NV, 9240 Zele (BE)
(72) Inventor: VAN INGELGEM, Werner, 9240 Zele (BE); SMET, Steven, 9240 Zele (BE); VAN MALDEREN, Bart, 9240 Zele (BE)
(74) Representative: D'Halleweyn, Nele Veerle Trees Gertrudis

(56) References cited:
- EP-A1- 2 450 012
- EP-A1- 3 167 858
- US-A1- 2017 079 858

## Description

### TECHNICAL FIELD

The present invention pertains to the technical field of absorbent articles, more preferably disposable personal care articles such as diapers, baby pants, adult incontinent garments, and the like, and to absorbent structures for use in such absorbent articles. More specifically the present invention relates to an absorbent structure comprising an absorbent core between a topsheet and a backsheet.

### BACKGROUND

Disposable absorbent articles have an absorbent structure for absorbing bodily exudates, a soft liquid pervious topsheet on the wearer side and a liquid impervious backsheet on the garment side. The absorbent structure in between is normally made from a mixture of cellulose fibers or other fibrous substance and an absorbent polymer material. These fibrous substances make these absorbent articles typically quite fluffy and bulky.

In recent years there has been increasing demand for flexible, thinner, lightweight absorbent structures to resolve various problems of manufacturing, marketing, design, fit, wearing comfort, distribution, garbage disposal, material and energy consumption, transportation and storage costs and the like.

The most common method currently used to meet these demands in disposable absorbent articles is to reduce the amount of cellulose fibre or other support material within and surrounding the absorbent structure and/or use larger amounts of absorbent polymer materials. Consequently such absorbent articles have a smaller proportion of hydrophilic cellulose fibres and/or a higher proportion of absorbent polymers materials. Some of these absorbent articles may be better at storing liquid, however they are not necessarily good at absorbing and distributing liquid when the absorbent article is actually being used. It will thus be apparent from the above that the absolute and relative proportions of the fibrous material and absorbent polymer material are closely linked in light of article performance.

In order to obtain good absorbency, distribution and retention within such absorbent structures it has found to be important to at least partially immobilize the absorbent material. Failing to provide sufficient structural integrity results in loss of functional performance characteristics such as coherence, absorption, distribution and/or retention and results in failures related but not limited to for instance leakages, high rewet values, etc.

EP 2 627 294 relates to a method and apparatus for forming a composite structure, preferably for use in an absorbent structure used within the personal hygiene industry, such as for instance feminine hygiene garments, baby diapers and pants and adult incontinence garments. The invention preferably provides a method and apparatus for depositing and positioning particulate materials in a desired pattern onto a moving carrier layer. The method allows accurate forming of a pattern of particulate material clusters at high production speed having improved attachment properties, with reduced raw material usage and relative low cost.

WO 2012/052173 relates to a method and apparatus for forming a composite structure, preferably for use in an absorbent structure used within the personal hygiene industry, such as for instance feminine hygiene garments, baby diapers and pants and adult incontinence garments. The method comprises depositing particulate material in a desired pattern onto a moving carrier layer and positioning it into a pocketing pattern. The method allows accurate forming of a pre-determined pattern of particulate material clusters at high production speed, with reduced raw material usage and relative low cost. As such method allows manufacturing of absorbent structures being substantially cellulose free and substantially glue free, considered technically, economically and environmentally friendly.

US 2017/0079858 A1 discloses an absorbent article, such as a diaper or a training pant, having a wearer-facing side and a garment-facing side and a longitudinal axis, includes an absorbent core with at least two longitudinally extending curved channels and an air-through bonded carded nonwoven layer as topsheet or acquisition layer under the topsheet.

EP2450012 A1 discloses an absorbent structure comprising a distribution layer having a minimum absorbent capacity, an immobilisation layer which joins to the distribution layer to define compartments there between, and an absorbent material held in at least one of the compartments.

There is a need in the art for an improved thin, flexible, lightweight absorbent structure which is discrete, sustainable and/or relatively inexpensive in view of manufacturing, marketing, design, fit, comfort, distribution, packaging, disposal, material, energy and transportation costs while preserving the required fluid absorption, distribution, transport, coherence and retention properties.

### SUMMARY

The object of embodiments of the invention is to provide an absorbent article of the type stated in the preamble, with reduced manufacturing cost, light weight, thin, and good liquid distribution and absorption capacities.

According to an aspect of the invention, there is provided an absorbent article according to claim 1.

By providing an absorbent core wherein the absorbent material is positioned directly between the bottom core wrap sheet and the liquid management structure, the absorbent material is adequately enclosed in the absorbent core without the need for a prior art top core wrap sheet. Moreover, by providing the liquid management structure to cover at least a surface portion of the absorbent material and to comprise at least one connecting portion attached to the bottom core wrap, a good immobilization of the absorbent material is acquired along with a strong structural integrity of the absorbent article. The at least one connecting portion forms at least one attachment zone capable of creating a channel for liquid distribution and absorption upon wetting. In this manner, at least one channel can be created with a reduced amount of material used for manufacturing the absorbent core, and as a result the manufacturing cost can be reduced while good liquid distribution and absorption capacities can be achieved. In addition, the thickness and weight of absorbent article may be reduced.

The liquid management structure is positioned directly between the absorbent material and the liquid pervious topsheet. In other words, between the liquid pervious topsheet and the absorbent material only the liquid management structure is present. This leads to an absorbent article with better and quicker absorption capacities as compared to prior art absorbent articles which have a liquid obstructing top core wrap positioned between the absorbent material and the liquid pervious topsheet or between the absorbent material and any liquid acquisition and/or liquid distribution layer(s) which may be present in the absorbent article. Moreover, by omitting the prior art top core wrap sheet, the absorbent article can be manufactured by using less raw material and thereby manufacturing costs can be reduced.

According to an embodiment the liquid management structure is essentially free of spunbond nonwoven material.

According to an embodiment the liquid management structure has a grammage of at least 20 g/m².

According to an embodiment the liquid management structure is in contact with an upper surface portion of the absorbent material. Because the liquid management structure is in contact with the absorbent material, liquid can efficiently and effectively be acquired and distributed by the absorbent core. As compared to prior art absorbent articles which comprise a top core wrap sheet which separates or shields the absorbent material from the top sheet or from any available liquid acquisition or distribution layer, liquid can be taken up by the absorbent material faster because the liquid management structure is in direct contact with an upper surface portion of the absorbent material.

According to an embodiment the liquid management structure extends over substantially an entire upper surface of the absorbent material. By extending over substantially an entire upper surface of the absorbent material, the liquid management structure provides for an optimal transition between the liquid pervious top sheet and the absorbent material of the absorbent core. Moreover, by extending over substantially an entire upper surface of the absorbent material, a good enclosure of the absorbent material in the absorbent core and a strong structural integrity is provided by the liquid management structure.

According to an embodiment, when seen from the top direction of the absorbent core, the liquid management structure has a total surface area of S1 and the absorbent core has a surface area of S0 defined by an area covered by the absorbent material plus an area of the at least one attachment zone, wherein S1 is smaller than 90% of S0.

According to an embodiment, S1 is smaller than 80% of S0, preferably S1 is smaller than 70% of S0, more preferably S1 is smaller than 60% of S0, even more preferably S1 is smaller than 50% of S0, most preferably S1 is smaller than 40% of S0.

According to an embodiment the at least one attachment zone is formed by heat-sealing the at least one connecting portion of the liquid management structure to the bottom core wrap sheet.

According to an embodiment the liquid management structure comprises a liquid acquisition layer.

According to an embodiment the liquid management structure comprises a liquid distribution layer.

According to an embodiment substantially no absorbent material is present in the at least one attachment zone between the liquid management structure and the bottom core wrap sheet.

According to an embodiment the at least one attachment zone extends from a crotch region in the direction of the first and/or second transverse edge of the absorbent core. Alternatively, or in addition, the at least one attachment zone extends in the direction from the first longitudinal edge to the second longitudinal edge of the absorbent core.

According to an embodiment the absorbent core is provided with at least a second attachment zone, said second attachment zone extending in the transversal direction of the absorbent core in between the first and second longitudinal edge.

According to an embodiment, the at least one attachment zone formed by the liquid management structure and the bottom core wrap comprises a permanent attachment, which is configured to remain attached when the absorbent material swells upon wetting.

According to an embodiment, the at least one attachment zone formed by the liquid management structure and the bottom core wrap comprises a semi-permanent attachment, which is configured to release when the absorbent material swells upon wetting.

According to an embodiment the at least one attachment zone is a continuous zone.

According to an embodiment the absorbent material comprises cellulosic fluff pulp and/or superabsorbent particles.

According to an embodiment the liquid management structure has a longitudinal dimension which is at least 20% of a length of the absorbent core, preferably at least 30%, more preferably at least 50%.

According to an embodiment the liquid management structure has a transverse dimension which is at least 5% of a width of the absorbent core, preferably at least 10%, more preferably at least 20%.

According to an embodiment a longitudinal dimension of the liquid management structure and the length of the absorbent core are within ±10% difference, and are preferably substantially the same.

According to an embodiment a transverse dimension of the liquid management structure and the width of the absorbent core are within ±10% difference, and are preferably substantially the same.

According to an embodiment the absorbent core comprises a second liquid management structure.

According to an embodiment a distance between the first and second liquid management structure is at least 5% of the width of the absorbent core.

According to an embodiment the at least one attachment zone comprises a first attachment zone and a second attachment zone.

According to an embodiment the first and second attachment zones extend next to each other from the crotch region in the direction of the first and/or the second transverse edge.

According to an embodiment the first and second attachment zones are connected through at least one semi-permanent attachment zone which preferably extends in a substantially transverse direction.

According to an embodiment the absorbent core comprises a bridge zone allowing a liquid flow between the first and the second longitudinal edge by capillary action through the absorbent material and/or by mass flow, such that upon wetting of the absorbent material, a front and rear channel are created, wherein the bridge zone extends between said front and rear channel; wherein a minimum distance between said front and rear channel is preferably larger than 3 mm more preferably larger than 5 mm

### BRIEF DESCRIPTION OF FIGURES

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figure 1A is a schematic cross-section of an exemplary embodiment of a diaper;
Figure 1B is a top plan view of the diaper of figure 1A;
Figure 2 is a schematic cross-section of an exemplary embodiment of a diaper;
Figure 3A is a schematic cross-section of an exemplary embodiment of a diaper;
Figure 3B is a top plan view of the diaper of figure 3A;
Figure 4 is a schematic cross-section of an exemplary embodiment of a diaper;
Figure 5A is a schematic cross-section of an exemplary embodiment of a diaper;
Figure 5B is a top plan view of the diaper of figure 5A
Figures 6A, 6B, 6C and 6D are top plan views of exemplary embodiments of a diaper;
Figure 7 is a top plan view of a further embodiment of a diaper;
Figure 8 is a top plan view of a further embodiment of a diaper;
Figure 9 is a top plan view of a further embodiment of a diaper; and
Figure 10 is a top plan view of a further embodiment of a diaper.

### DESCRIPTION OF EMBODIMENTS

As used herein, the following terms have the following meanings:
"A", "an", and "the" as used herein refers to both singular and plural referents unless the context clearly dictates otherwise. By way of example, "an edge barrier" refers to one or more than one edge barrier.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent core" as used herein refers to a three-dimensional part of the absorbent structure, comprising liquid-absorbing material, useful to permanently absorb and/or retain bodily exudates.
"Absorbent component" as used herein refers to a structural constituent of an absorbent article, e.g., a piece of an absorbent core, such as one of multiple pieces in a multi-piece absorbent core.
"Absorbent element" as used herein refers to a part of a functional constituent of an absorbent structure, e.g., a acquisition layer, a dispersion layer, core layer or a release structure formed of a material or materials having particular liquid handling characteristics suitable for the specific function.
"Absorbent fibrous polymer material" as used herein refers to an absorbent polymer material which is in threadlike from such as fibers, filaments, and the like so as to be less flowable in the dry state than particulates.
"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g. bond area's) or unintentional (e.g. manufacturing artifacts).
"Absorbent particulate polymer material" as used herein refers to an absorbent polymer material which is in particulate form such as powders, granules, flakes and the like so as to be flowable in the dry state.
"Absorption" as used herein refers to the process by which a liquid is taken up within a material.
"Absorption rate" as used herein refers to the rate of absorption of liquid, i.e. the amount of liquid which is absorbed per unit of time, typically by an absorbent component, element and/or absorbent layer of the absorbent article, structure and/or core.
"Acquisition layer", "acquisition region", "acquisition surface" or "acquisition material" and the like as used herein refer to the layer overlying the absorbent core and/or absorbent material having a faster liquid uptake and/or distribution capability.
"Absorbency" is the ability of a material to take up fluids by various means including capillary, osmotic, solvent, chemical and/or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".
"Apparent density", "density" as used herein refers to the basis weight of the sample divided by the caliper with appropriate unit conversions incorporated therein. Apparent density used herein has the unit g/cm³.
"Attach", "attached" and "attachment" as used herein are synonymous with their counterparts of the terms "fasten", "affix", "secure", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Back region" as used herein refers to the portion of an absorbent article or part thereof that is intended to be positioned proximate the back of a wearer.
"Backing" as used herein refers to a web or other material that supports and reinforces the back of a product.
"Basis weight" is the weight per unit area of a sample reported in grams per square meter, g/m² or gsm.
"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.
"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibers" as used herein refers to naturally occurring fibers based on cellulose, such as, for example cotton, linen, etc; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers.
"Cluster" or the like as used herein refers to an agglomeration of particles and/or fibers.
"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc), altering the chemical structure of the fibers themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.
"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them in soluble.
"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Dispersion layer", "dispersion region", "dispersion surface" or "dispersion material" and the like as used herein refer to the layer overlying the absorbent core having a faster liquid uptake and dispersion capability.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".
"Dry strength" as used herein refers to the strength of ajoint determined in dry state conditions, immediately after drying under specified conditions or after a period of conditioning in the standard laboratory atmosphere.
"Essentially cellulose free" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.
"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.
"Fabric" as used herein refers to a sheet structure made from fibers, filaments and/or yarns.
"Feminine hygiene garments" as used herein refer to absorbent hygiene articles intended to be worn by woman, for absorbing and containing body exudates.
"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.
"Fluff pulp" or "Pulp fluff" as used herein refers to wood pulp specially prepared to be drylaid. The fibers can be either natural or synthetic or a combination thereof.
"Front region" as used herein refers to the portion of an absorbent article or part thereof that is intended to be positioned proximate the front of a wearer.
"Garment facing layer" as used herein refers to elements of the chassis that form the outer surface of the absorbent article, such as the backsheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"High loft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilization layer" as used herein refers to a layer able to be applied to the absorbent polymer material or absorbent polymer material area with the intent to gather, bond and/or immobilize absorbent material and/or absorbent layer.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibers with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered."
"Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration.
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the f low of a liquid f rom one absorbent element or component to another absorbent element or component by channel flow action.
"Mechanical bonding" as used herein refers to a method of bonding fibers by entangling them. This can be achieved by needling, stitching with fibers or by the use of high-pressure air or water jets and the like.
"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).
"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or nonrefastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back of the wearer.
"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.
"Resin" as used herein refers to a solid or semisolid polymeric material.
"Thermobonding" as used herein refers to a method of bonding fibers by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localized heat through vibration thereby causing thermoplastic fibers to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibers produced by weaving is herein referred to as a "woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
"Wet strength" as used herein refers to the strength of a joint determined immediately after removal from a liquid in which it has been immersed under specified conditions of time, temperature and pressure. The term is commonly used in the art to designate strength after immersion in water.
"Wetlaying" as used herein refers to the forming a web from an aqueous dispersion of fibers by applying modified paper making techniques; a web of fibers produced by wetlaying is herein referred to as a "wetlaid".
"Wood pulp" as used herein refers to cellulosic fibers used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the width and length, respectively, of the article, structure or element.
"Z-dimension" as used herein refers to the dimension orthogonal to the length and width of the article, structure or element. The z-dimension corresponds generally to the thickness of the article, structure or element.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The same or similar features and components are indicated with the same reference numerals throughout the figures.

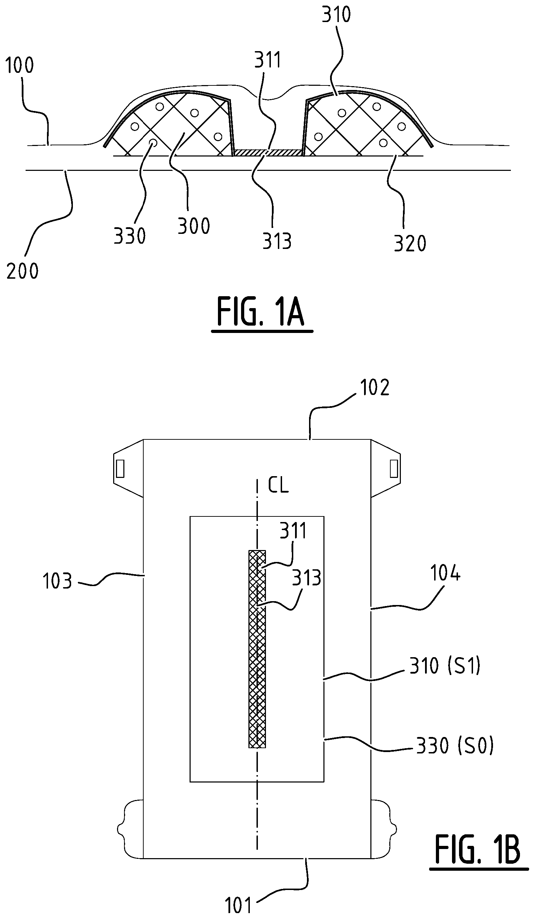

Figures 1A and 1B illustrate an exemplary embodiment of an absorbent article, here a diaper. Figure 1A shows a cross-section of the absorbent article, and Figure 1B shows the absorbent article in its flat out, un-contracted state with the wearer side facing the viewer. The skilled person understands that the absorbent article may also be a pant or an adult incontinence garment or the like. The absorbent article comprising a liquid pervious topsheet 100, a liquid impervious backsheet 200, and an absorbent core 300 positioned between the liquid pervious topsheet 100 and the liquid impervious backsheet 200. In a possible embodiment the absorbent article may further comprise adhesive between the absorbent core 300 and the liquid pervious topsheet 100. The absorbent article has a first and second longitudinal edge 103, 104 and a first and second transverse edge 101, 102. The absorbent core 300 comprises a bottom core wrap sheet 320, absorbent material 330, and a liquid management structure 310. The absorbent material 330 is positioned directly between the bottom core wrap sheet 320 and the liquid management structure 310. There is no prior art top core wrap sheet present. In the illustrated embodiment, the liquid management structure 310 has a surface S1 which covers an entire upper surface S0 of the absorbent material 330 which may corresponds with an upper surface of the absorbent core 300. In this manner, the liquid management structure can perform the task of physically containing the absorbent material 330 in place, which task in prior art absorbent articles is performed by a dedicated top core wrap sheet. In addition to this functionality, the liquid management structure 310 performs the task of acquiring and/or distributing liquid, which travels through the liquid pervious topsheet 100, to the absorbent material 330. The liquid management structure 310 comprises at least one connecting portion 311 which is attached to the bottom core wrap sheet 320 forming at least one attachment zone 313. The attachment zone 313 extends from the crotch region in the direction of the first and second transverse edge 101, 102. Upon wetting op the absorbent material 330 a channel is created at the attachment zone 313. In this manner liquid can be efficiently distributed to the absorbent material 330. By providing a liquid management structure 310 such that the absorbent material 330 is positioned directly between the bottom core wrap sheet 320 and the liquid management structure 310, the provision of a dedicated top core wrap sheet, as seen in prior art absorbent articles, can be omitted. This allows the amount of raw material for manufacturing the absorbent article to be reduced. As a result the manufacturing costs are reduced. At the same time, by providing the liquid management structure 310 with the above described functionality, an absorbent article with good liquid distribution and absorption capacities is obtained.

The liquid management structure 310 is positioned directly between the absorbent material 330 and the liquid pervious topsheet 100. In such an embodiment, only the liquid managing structure 310 is present between the topsheet and the absorbent material. In alternative embodiments, adhesive may be present between the absorbent material 330 and the liquid pervious topsheet 100 , e.g. between the topsheet 100 and the liquid management structure 310.

The illustrated liquid management structure 310 is preferably essentially free of spunbond nonwoven material. More preferably, the liquid management structure 310 is essentially free of spunbond nonwoven material with a grammage or basis weight smaller than 15 g/m². These materials are considered to have poor liquid acquisition and/or distribution capacities. The liquid management structure 310 has a grammage of at least 19 g/m², preferably at least 20 g/m². Carded nonwoven materials with a grammage of at least 15g/m² are preferred. The liquid management structure 310 comprises meltblown nonwoven material and/or spunbond nonwoven material with a grammage of at least 15g/m². This may contribute to the liquid acquisition and/or distribution capacities of the absorbent article. Moreover, the liquid management structure 310 may be made of single substrate, material or nonwoven, or may comprise two or more substrates, materials or nonwovens. Preferably, the liquid management structure 310 is in contact with a surface portion of the absorbent material 330. This improves the transition and distribution of the liquid between the liquid management structure 310 and the absorbent material.
The liquid management structure 310 may comprise an acquisition layer. In addition, or alternatively, the liquid management structure 310 may comprise a distribution layer.

The acquisition layer may comprise a nonwoven material comprising a carded nonwoven or a spunbond nonwoven material with a grammage of at least 15g/m². The acquisition layer may comprise a combination of synthetic and cellulosic fibers which are bonded together to form a highloft material. The skilled person is aware of alternative acquisition layers.
The distribution layer may comprise cross-linked cellulose fibers, which may be twisted, curled, and/or crimped. The skilled person is aware of alternative distribution layers.
In an exemplary embodiment, the liquid management structure 310 comprises a textile structure with super absorbent particles blended therein, preferably in a homogeneous manner. In other words, the liquid management structure may comprise a super absorbent particles impregnated highloft structure. The liquid management structure 310 may be bonded to the absorbent material 330 by means of gluing, ultrasonic bonding, thermobonding, and the like. In an alternative embodiment no additional bonding between the liquid management structure 310 and the absorbent material 330 is carried out.

Seen in a top view of the absorbent core 300, as illustrated in Figure 1B, the liquid management structure 310 has a total surface area of S1, and the absorbent core 300 comprising the absorbent material 330 has a surface area of S0 defined by an area covered by the absorbent material 330 plus an area of the attachment zone 313. In the illustrated embodiment, S1 is substantially equal to S0 such that the liquid management structure 310 extends over the entire upper surface of the absorbent core 300. However, in alternative embodiments S1 may be smaller than S0 as will be described later in this text. By providing the liquid management structure 310 having a total surface area S 1 which is smaller than S0, the amount of raw material used for manufacturing the absorbent article can be reduced even more. In alternative embodiments, S1 may be larger than S0.

The attachment zone 313 has a center line CL, which is a straight line. In other embodiments, the center line CL may be a curve, a polyline, or any other shape. The center line CL is defined as a line which is at the same distance of opposite edges of the attachment zone 313, and which preferably extends in a length direction of the attachment zone 313. The attachment zone 313 extends from a crotch region in the direction of the first and second transverse edge 101, 102 of the absorbent article which allows a better liquid distribution between the crotch region and a front/back portion of the absorbent article. In other embodiments, the at least one attachment zone 313 may extend in the direction from the first longitudinal edge 103 to the second longitudinal edge 104 of the absorbent article, thereby allowing a better liquid distribution between left and right portions of the absorbent article. In a further embodiment, it is also possible for the at least one attachment zone 313 to extend under a small angle with respect to the longitudinal direction of absorbent core 300, e.g. an angle between 5 and 10°. Preferably the at least one attachment zone 313 is arranged symmetrically with respect to a longitudinal center line of absorbent core 300. A contour of the attachment zone 313 is adjacent to the absorbent material, which may comprise cellulosic fluff pulp and/or superabsorbent particles. A length of the attachment zone 313 is larger than 10% of the length of the absorbent core 300, more preferably larger than 30%, even more preferably larger than 50%, which allows a better liquid distribution over a larger area of the absorbent core 300. The attachment zone 313 may be a permanent attachment zone which remains attached when wetted, allowing the channel to distribute liquid during consecutive liquid insults.

The liquid managing structure 310 may have a substantially rectangular shape. In various embodiments, the liquid managing structure 310 may have a longitudinal dimension which ranges from 50% to substantially 100% of the length of the absorbent core 300, and a transverse dimension which ranges from 50% to substantially 100% of the width of the absorbent core 300. In the illustrated embodiment of Figure 1B, the longitudinal dimension and the transverse dimension of the liquid managing structure 310 are substantially 100% of the length and the width of the absorbent core 300, respectively. A rear and/or front edge of the liquid managing structure 310 may be attached to a corresponding rear and front edge of the bottom core wrap sheet 320, respectively, to provide a stable and integrated structure of the absorbent core 300 while the use of material is reduced. Alternatively, or in addition, a left side and/or right side edge of the liquid managing structure 310 may be attached to a corresponding left side and right side edge of the bottom core wrap sheet 320, respectively.

Preferably the attachment between liquid management structure 310 and bottom core wrap sheet 320 is a permanent attachment, and the absorbent core 300 is configured such that, in a wetted state of absorbent core 300, the absorbent material 330 may extend partially over a bottom of the channel. Alternatively, the attachment between liquid managing structure 310 and bottom core wrap sheet 320 may be a semi-permanent attachment configured to release after having been in contact with urine for a predetermined period of time, and the predetermined period of time is preferably smaller than 30 seconds.

The attachment zone 313 is provided by means of continuous attachments in the longitudinal direction of the absorbent core 300 in Figures 1A and 1B. It is however clear to the skilled person that in a possible embodiment the attachment zone may be provided by means of continuous attachments in the transversal direction of the absorbent core 300 and/or discontinuous attachments in the transversal direction of the absorbent core 300 and/or discontinuous attachments in the longitudinal direction of the absorbent core 300. In an embodiment the at least one attachment zone 313 is formed by heat-sealing the at least one portion of the liquid management structure 310 to the bottom core wrap sheet 320. Alternatively, or in addition, the at least one attachment zone 313 may be formed by means of any one of the following or a combination thereof: pressure bonding, thermobonding, sonic bonding, chemical bonding, adhesive, mechanical bonding.

In a further embodiment, the channel created by the attachment zone 313 may be indicated with a color and/or with a pattern which is different from the color and/or pattern of the topsheet 100 and/or backsheet 200. More in particular the area of the channel may comprise a print allowing a user to visually distinguish the at least one channel. This print may be arranged on the topsheet 100, on the liquid managing structure 310, on the bottom core wrap sheet 320, on the backsheet 200, or on any sheet in between the topsheet 100 and the backsheet 200, as long as it is visible for a user. As the sheets may be partially transparent, the print may be arranged on a sheet in between the topsheet 100 and the backsheet 200, as long as it is visible through the topsheet 100 and/or the backsheet 200. Preferably the print is visible when looking at the topsheet 100 of the absorbent article.

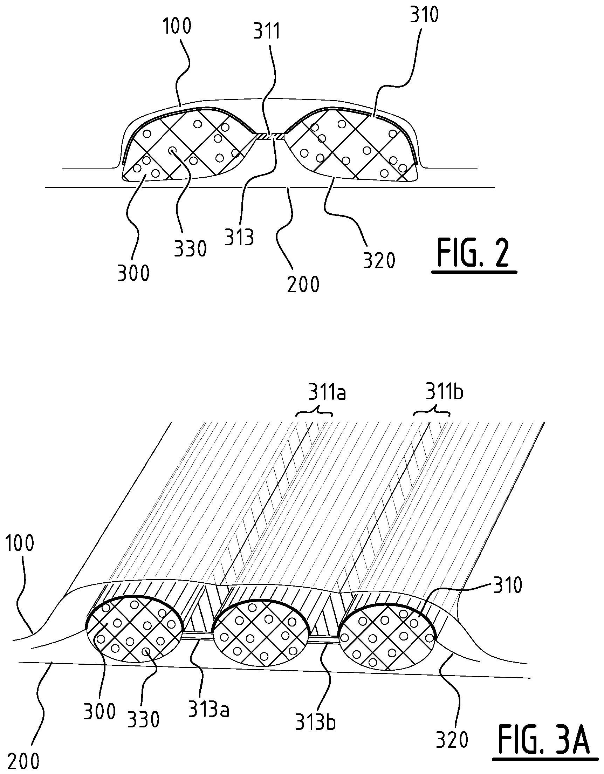

Figure 2 shows a cross-section of an absorbent article according to an embodiment, which is similar to the embodiment as illustrated in Figures 1A and 1B. However, whereas in the embodiment of Figures 1A and 1B, the connecting portion 311 of the liquid management structure 310 is attached to the bottom core wrap sheet 320 at the bottom of the absorbent core 300, in the embodiment of Figure 2 the connecting portion 311 of the liquid management structure 310 is attached to the bottom core wrap sheet 320 at a location midway the absorbent core 300. In other words, the attachment zone 313 in Figure 1A is located close to the backsheet 200, at the bottom of the absorbent core 300, whereas the attachment zone 313 in Figure 2 is located further away from the backsheet 200, in the middle of the thickness of the absorbent core 300. In this manner, air channels may be created in between the back sheet 200 and the absorbent core 300 at the location of the attachment zone 313. It is clear to the skilled person that the attachment of the portion 311 of the liquid management structure 310 to the bottom core wrap sheet 320 can be positioned anywhere along the thickness dimension of the absorbent core 300. Preferably, in the at least one attachment zone 313 substantially no absorbent material 330 is present between the liquid management structure 310 and the bottom core wrap sheet 320.

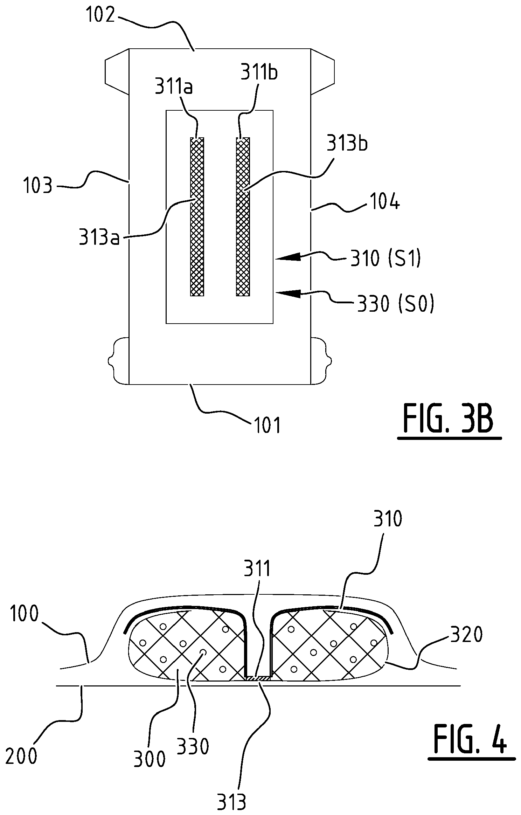

Figures 3A and 3B illustrate an exemplary embodiment of an absorbent article, here a diaper. Figure 3A shows a cross-section of the absorbent article, and Figure 3B shows the absorbent article in its flat out, un-contracted state with the wearer side facing the viewer. Whereas embodiments of absorbent articles according to the previous figures comprise one liquid management structure 310 with one connection portion 311 and one corresponding attachment zone 313, the embodiment according to Figures 3A and 3B illustrate one liquid management structure 310 comprising two connection portions 311a, 311b which are attached to the bottom core wrap sheet 320 to form two corresponding attachment zones 313a, 313b. More in particular, Figure 3A illustrates an absorbent article comprising a liquid pervious topsheet 100, a liquid impervious backsheet 200, and an absorbent core 300 positioned between the liquid pervious topsheet 100 and the liquid impervious backsheet 200. As shown in Figure 3B, the absorbent article has a first and second longitudinal edge 103, 104 and a first and second transverse edge 101, 102. The absorbent core 300 comprises a bottom core wrap sheet 320, absorbent material 330, and a liquid management structure 310. The absorbent material 330 is positioned directly between the bottom core wrap sheet 320 and the liquid management structure 310. The surface area S1 of the liquid management structure 310 covers substantially an entire upper surface area S0 of the absorbent material 330. The liquid managing structure 310 comprises two connecting portions 311a, 311b which are attached to the bottom core wrap sheet 320 forming two corresponding attachment zones 313a, 313b. In the illustrated embodiment, the two attachment zones 313a, 313b, extend next to each other from the crotch region in the direction of the first and the second transverse edge 101, 102. It is clear to the skilled person that more than two attachment zones may be provided and that the orientation, shape and/or dimensions of the one, two or more attachment zones may vary. In a further embodiment, the first and second attachment zones 313a, 313b are connected through at least one additional attachment zone (not illustrated) which preferably extends in a substantially transverse direction. Preferably the at least one additional attachment zone (not illustrated) is a semi-permanent attachment zone.

Figure 4 shows a cross-section of an absorbent article according to an embodiment, which is similar to the embodiment as illustrated in Figures 1A and 1B. In the embodiment of Figures 1A and 1B, the bottom core wrap sheet 320 is substantially flat and covers the bottom of the absorbent core 300 whereas the liquid management structure 310 covers the upper portion of the absorbent core 300 as well as side portions thereof such that the liquid management structure 310 and the bottom core wrap sheet 320 enclose the absorbent core 300. In the embodiment of Figure 2 the liquid management structure 310 covers the upper portion of the absorbent core 300, whereas the bottom core wrap sheet 320 covers the bottom of the absorbent core, as well as side portions thereof such that the liquid management structure 310 and the bottom core wrap sheet 320 enclose the absorbent core 300. Moreover, the liquid management structure 310 partially overlaps with the bottom core wrap sheet 320 at the side edges of the upper portion of the absorbent core 300.

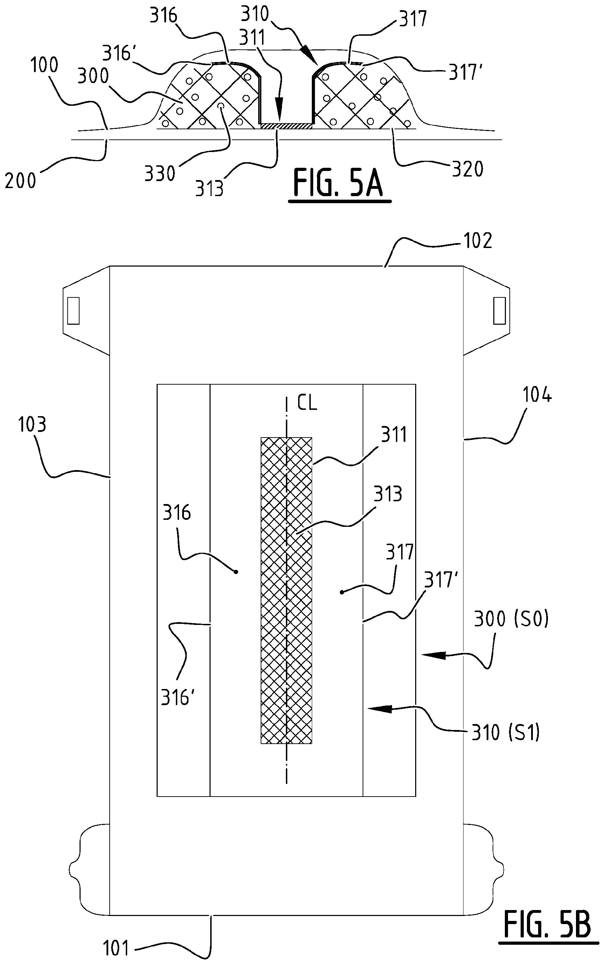

Figures 5A and 5B illustrate an exemplary embodiment of an absorbent article, here a diaper. Figure 5A shows a cross-section of the absorbent article, and Figure 5B shows the absorbent article in its flat out, un-contracted state with the wearer side facing the viewer. The absorbent article comprises a liquid pervious topsheet 100, a liquid impervious backsheet 200, and an absorbent core 300 positioned between the liquid pervious topsheet 100 and the liquid impervious backsheet 200. The absorbent article has a first and second longitudinal edge 103, 104 and a first and second transverse edge 101, 102.

The absorbent core 300 comprises a liquid management structure 310, a bottom core wrap sheet 320, and absorbent material 330 arranged partially between the liquid management structure 310 and the bottom core wrap sheet 320. The liquid management structure 310 comprises a connecting portion 311 which is attached to the bottom core wrap sheet 320 forming an attachment zone 313. The liquid management structure 310 further comprises edge portions 316, 317 covering a portion of the absorbent material 330. Each edge portion 316, 317 has a free edge 316', 317', wherein the free edges 316', 317' are not connected to the bottom core wrap sheet 320. The first edge portion 316 and the second edge portion 317 are located at opposite sides of the attachment portion 311. In this manner the first edge portion 316 and the second edge portion 317 provide a stable structural basis for the formation of an attachment zone 313 and a corresponding channel. When the absorbent core is wetted, the absorbent material 330 swells such that the first edge portion 316 and the second edge portion 317 form embankments delimiting the channel for guiding the liquid. A portion of the absorbent material 330 is not covered by the liquid management structure 310. In a possible embodiment the liquid management structure 310 may be attached to the liquid pervious topsheet 100, e.g. using adhesive. The attachment zone 313 extends from the crotch region CR in the direction of the first and second transverse edge 101, 102. Upon wetting of the absorbent material 330, a channel is created at said attachment zone 313. The absorbent material 330 may swell upon wetting, and the edge portions 316, 317 may prevent the absorbent material 330 from entering the attachment zone 313 and help with formation of embankments delimiting the channel upon wetting. This embodiment allows the amount of raw material for manufacturing the liquid management structure 310 to be reduced, and as a result the manufacturing cost can be reduced. Meanwhile an absorbent article with good liquid distribution and absorption capacities can still be obtained.

Seen in a top view of the absorbent core 300, the liquid management structure 310 has a total surface area of S1 and the absorbent core 300 has a surface area of S0 defined by an area covered by the absorbent material 330 plus an area of the at least one attachment zone 313. S1 is smaller than 90% of S0, preferably S1 is smaller than 80% of S0, more preferably S1 is smaller than 70% of S0, even more preferably S1 is smaller than 60% of S0, even more preferably S1 is smaller than 50% of S0, most preferably S1 is smaller than 40% of S0.

The attachment zone 313 has a center line CL, which is a straight line. In other embodiments, the center line CL may be a curve, a polyline, or any other shape. The center line is a line which is at the same distance of opposite edges of the attachment zone 313, and which preferably extends in a length direction of the attachment zone 313. The attachment zone 313 extends from a crotch region in the direction of the first and second transverse edge of the absorbent article 101, 102, which allows a better liquid distribution between crotch region and front/back portion of absorbent article. In other embodiments, the at least one attachment zone 313 may extend in the direction from the first longitudinal edge 103 to the second longitudinal edge 104 of the absorbent article, which allows a better liquid distribution between left and right portions of absorbent article. However, it is also possible for the at least one attachment zone 313 to extend under a small angle with respect to the longitudinal direction of absorbent core 300, e.g. an angle between 5 and 10°. Preferably the at least one attachment zone 313 is arranged symmetrically with respect to a longitudinal center line of absorbent core 300. A contour of the attachment zone 313 is adjacent to the absorbent material, which may comprise cellulosic fluff pulp and/or superabsorbent particles. A length of the attachment zone 313 is larger than 10% of the length of the absorbent core 300, more preferably larger than 30%, even more preferably larger than 50%, which allows a better liquid distribution over a larger area of the absorbent core 300. The attachment zone 313 may be a permanent attachment zone which remains attached when wetted, allowing the channel to distribute liquid during consecutive liquid insults.

The liquid management structure 310 has a substantially rectangular shape. The liquid management structure 310 has a longitudinal dimension which is substantially 100% of the length of the absorbent core 300 and a transverse dimension which is about 60% of the width of the absorbent core 300. In this embodiment, a rear and front edge of the liquid management structure 310 may be attached to a rear and front edge of the bottom core wrap sheet 320, respectively, to provide a stable and integrated structure of the absorbent core 300 while the use of material can still be reduced. It is clear to the skilled person that the ratio of the longitudinal dimension of the liquid management structure 310 to the length of the absorbent core 300 may vary. It is further clear to the skilled person that the ratio of the transverse dimension of the liquid management structure 310 to the width of the absorbent core 300 may vary. In various embodiments, the liquid managing structure 310 may have a longitudinal dimension which ranges from 50% to substantially 100% of the length of the absorbent core 300, and/or a transverse dimension which ranges from 50% to substantially 100% of the width of the absorbent core 300.

In other embodiments, a transverse dimension of the liquid management structure 310 and/or a transverse dimension of the bottom core wrap sheet 320 and the width of the absorbent core 300 may be within ±10% difference, preferably substantially the same, which allows an attachment between the liquid management structure 310 and the bottom core wrap sheet 320 by the longitudinal edges of the absorbent core 300.

Preferably the at least one attachment between the liquid management structure 310 and bottom core wrap sheet 320 may be a permanent attachment, wherein the absorbent core 300 is configured such that, in a wetted state of absorbent core 300, the absorbent material 330 may extend partially over a bottom of the channel formed by the attachment. Alternatively, the attachment between the liquid management structure 310 and bottom core wrap sheet 320 may be a semi-permanent attachment configured to release after having been in contact with urine for a predetermined period of time, and the predetermined period of time is preferably smaller than 30 seconds.

The attachment zone 313 is provided by means of continuous attachments in the longitudinal direction of the absorbent core in Figure 5A and 5B. It is clear to the skilled person that in a possible embodiment the attachment zone, or any additional attachment zone, may be provided by means of continuous attachments in the transversal direction of the absorbent core and/or discontinuous attachments in the transversal direction of the absorbent core and/or discontinuous attachments in the longitudinal direction of the absorbent core.

The illustrated liquid management structure 310 is preferably essentially free of spunbond nonwoven material. More preferably, the liquid management structure 310 is essentially free of spunbond nonwoven material with a grammage or basis weight smaller than 15 g/m². These materials are considered to have poor liquid acquisition and/or distribution capacities in comparison with nonwoven materials with a higher basis weight. The liquid management structure 310 has a grammage of at least 19 g/m², preferably at least 20 g/m². Carded nonwoven materials with a grammage of at least 15g/m² are preferred. The liquid management structure 310 comprises meltblown nonwoven material and/or spunbond nonwoven material with a grammage of at least 15g/m². This contributes to the liquid acquisition and/or distribution capacities of the absorbent article. Moreover, the liquid management structure 310 may be made of single substrate, material or nonwoven, or may comprise two or more substrates, materials or nonwovens. Preferably, the liquid management structure 310 is in contact with a surface portion of the absorbent material 330. This improves the transition and distribution of the liquid between the liquid management structure 310 and the absorbent material.

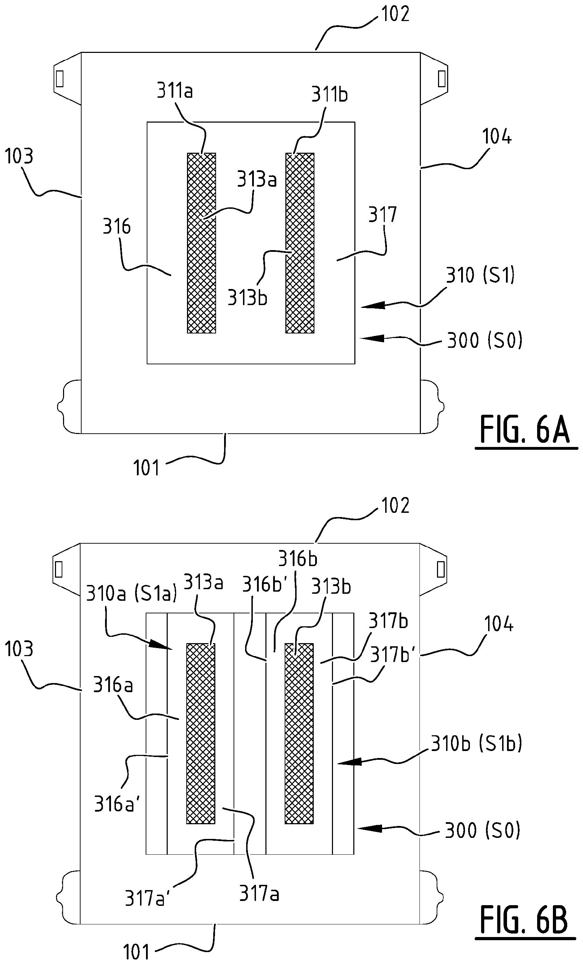

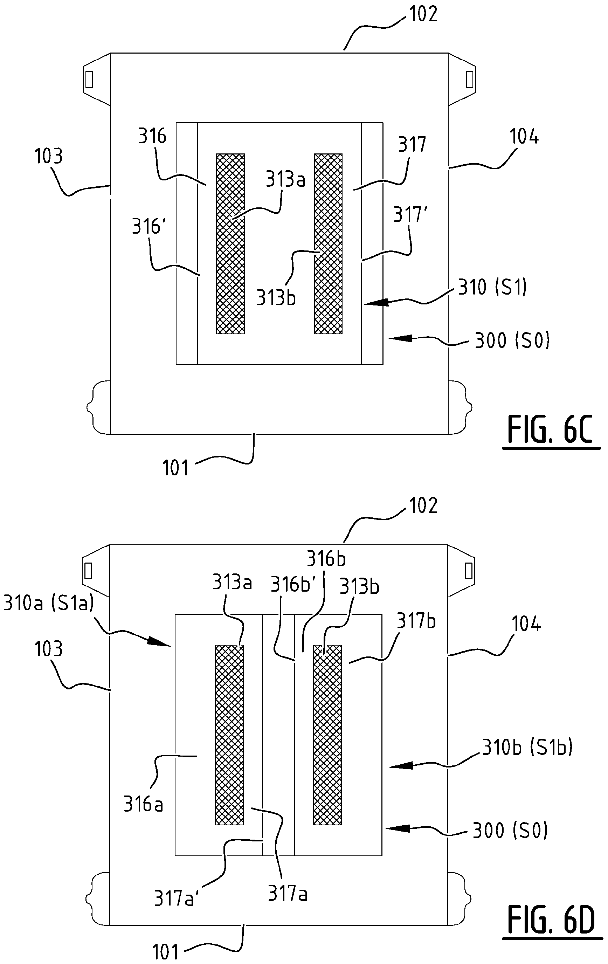

Figures 6A, 6B, 6C and 6D are top plan views of exemplary embodiments of an absorbent article, wherein Figures 6A and 6C illustrate embodiments comprising one liquid management structure 310 having two attachment zones 313a, 313b, and Figures 6B and 6D illustrate embodiments comprising two liquid management structures 310a, 310b, wherein each liquid management structure 310a, 310b has one corresponding attachment zone 313a, 313b.
The embodiment of Figure 6A corresponds to the embodiment as previously described in view of Figures 3A and 3B. In Figure 6A edge portions 316 and 317 of the liquid management structure 310 are indicated which do not have free edges. Since the liquid management structure 310 has similar dimensions as compared to the absorbent core 300, more in particular since S1 is substantially equal to S0, the liquid management structure 310 can be connected to the bottom core wrap sheet 320 at the longitudinal edges of the edge portions 316 and 317.
In Figure 6C the total surface area S1 of the liquid management structure 310 is smaller than the surface area S0 of the absorbent core 300. More in particular, the total surface area S1 of the liquid management structure 310 is smaller than 90% of the surface area S0 of the absorbent core 300. It can be seen in Figure 6C that edge portions 316 and 317 have free edges 316'and 317', respectively, in the longitudinal direction of the absorbent core.
Figure 6B illustrates an embodiment comprising two liquid management structures 310a, 310b, wherein each liquid management structure 310a, 310b has one corresponding attachment zone 313a, 313b and has two edge portions 316a, 317a, 316b, 317b with corresponding free edges 316a', 317a', 316b', 317b'. Seen in a top view of the absorbent core 300, the first liquid management structure 310a has a total surface area of S1a, the second liquid management structure 310b has a total surface area of S1b, and the absorbent core 300 has a surface area of S0 defined by an area covered by the absorbent material 330 plus an area of the attachment zones 313a, 313b. S1a and S1b may be similar, preferably substantially the same, and S1a+S1b is smaller than 90% of S0, preferably 80% of S0, more preferably 70% of S0, and most preferably 60% of S0. The distance between the liquid management structure 310a and the second liquid management structure 310b may be at least 5% of the width of the absorbent core 300.
Figure 6D illustrates an embodiment comprising two liquid management structures 310a, 310b, wherein each liquid management structure 310a, 310b has one corresponding attachment zone 313a, 313b and has two edge portions 316a, 317a, 316b, 317b wherein edge portions 317a, 316b have corresponding free edges 317a', 316b' and edge portions 316a, 317b have corresponding longitudinal edges which may be connected to the bottom core wrap sheet 320. Seen in a top view of the absorbent core 300, the first liquid management structure 310a has a total surface area of S1a, the second liquid management structure 310b has a total surface area of S1b, and the absorbent core 300 has a surface area of S0 defined by an area covered by the absorbent material 330 plus an area of the attachment zones 313a, 313b. S1a and S1b may be similar, preferably substantially the same, and S1a+S1b is smaller than 90% of S0, preferably 80% of S0, more preferably 70% of S0, and most preferably 60% of S0. The distance between the liquid management structure 310a and the second liquid management structure 310b may be at least 5% of the width of the absorbent core 300.
It is clear to the skilled person that considerations made above in view of the transverse dimension of the liquid management structure 310, 310a, 310b being smaller than the width, or than a percentage of the width, of the absorbent core 300, can alternatively or in addition apply to the longitudinal dimension of the liquid management structure 310, 310a, 310b being smaller than the length, or than a percentage of the length of the absorbent core 300, mutatis mutandis.
It is further clear to the skilled person that alternatively or in addition to multiple liquid management structures 310a, 310b being positioned next to each other with a transverse distance between them, multiple liquid management structures may be positioned in front or behind one another with a longitudinal distance between them.

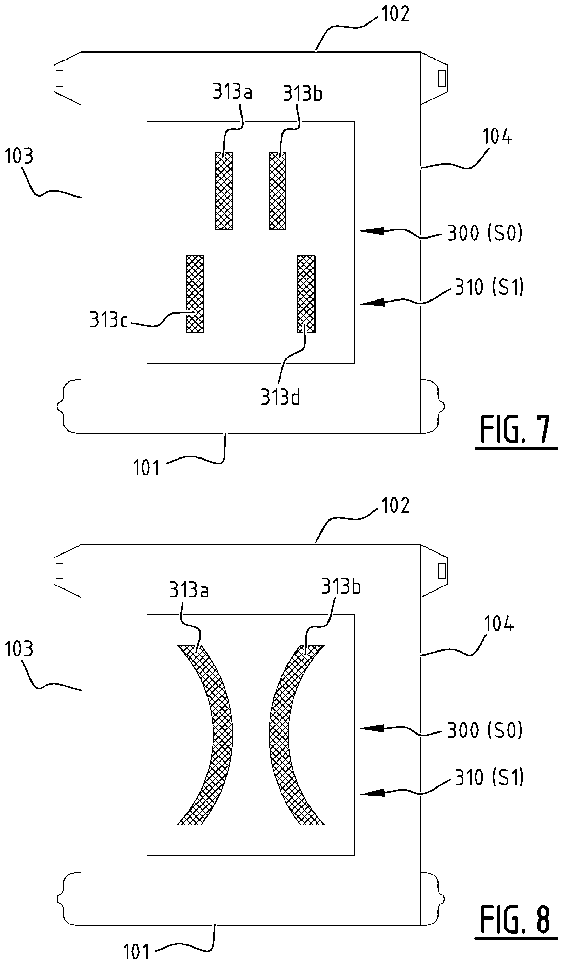

Figure 7 shows an exemplary absorbent article in its flat out, un-contracted state with the wearer side facing the viewer. The skilled person understands that the absorbent article may also be a pant or an adult incontinence garment or the like. The absorbent article comprises a liquid pervious topsheet, a liquid impervious backsheet, and an absorbent core 300 positioned in between the topsheet and the backsheet. The absorbent core 300 comprises absorbent material 330 between a liquid management structure 310 and a bottom core wrap sheet 320. The absorbent article has a first and second longitudinal edge 103, 104 and a first and second transverse edge 101, 102.

The absorbent core 300 is provided with a plurality of attachment zones 313a, 313b, 313c, 313d comprising at least a first attachment zone 313a and a second attachment zone 313b. The first and second attachment zones extend next to each other from the crotch region CR in the direction of the first and/or second transverse edge 101, 102. In first and second attachment zone 313a, 313b the liquid management structure 310 is attached to the bottom core wrap sheet 320
- along an attachment which extends, seen in a transverse direction of the absorbent core, over a transverse distance which is at least 1 mm, preferably at least 2 mm, more preferably at least 3mm, most preferably at least 4mm; and/or
- along a discontinuous attachment at a plurality of locations at a distance of each other, seen in the transverse direction of the absorbent core. In that manner, upon wetting of the absorbent material, a first and second channel are created at said first and second attachment zone 313a, 313b, respectively.

First attachment zone 313a and second attachment zone 313b are substantially parallel and run in the longitudinal direction of absorbent core 300. However, it is also possible for first and second attachment zone 313a, 313b to extend under a small angle with respect to the longitudinal direction of absorbent core 300, e.g. an angle between 5 and 10°. For example, first and second attachment zone 313a, 313b may be diverging slightly outwardly in the direction of second transverse edge 102. Preferably first attachment zone 313a and second attachment zone 313b are arranged symmetrically with respect to a longitudinal center line CL of absorbent core 300.

The absorbent article is further provided with a third and a fourth attachment zone 313c, 313d located at a distance each other. Third and fourth attachment zone 313c, 313d each extend from the crotch region in the direction of first transverse edge 101. The distance between first and second attachment zone 313a, 313b may be different from the distance between third and fourth attachment zone 313c, 313d.

Third attachment zone 313c and fourth attachment zone 313d are substantially parallel and run in the longitudinal direction of absorbent core 300. However, it is also possible for third and fourth attachment zone 313c, 313d to extend under a small angle with respect to the longitudinal direction of absorbent core 300, e.g. an angle between 5 and 10°. For example, third and fourth attachment zone 313c, 313d may be diverging slightly outwardly in the direction of first transverse edge 101. Preferably third attachment zone 313c and fourth attachment zone 313d are arranged symmetrically with respect to a longitudinal center line CL of absorbent core 300.

In the embodiment of Figure 7 only one liquid management structure 310 is provided which comprises four attachment zones 313a, 313b, 313c, 313d and which comprises no free edges. In other words, seen from the top direction of the absorbent core, the liquid management structure has a total surface area of S1 which is substantially equal to S0 which is the surface area of the absorbent core defined by an area covered by the absorbent material plus an area of the four attachment zones 313a, 313b, 313c, 313d. It is however clear to the skilled person that a similar pattern of attachment zones 313a, 313b, 313c, 313d may be provided by 1, 2, 3 or 4 separate liquid managing structures, wherein each liquid managing structure may haves none, 1, 2, 3 or 4 free edges, wherein the total combined surface area S1 of the liquid managing structure(s) is smaller than 90% of S0 (see Figures 6A-6D).

In the embodiment of Figure 8, the absorbent core 300 comprises a liquid management structure 310, a bottom core wrap sheet 320 (not shown), and absorbent material 330 arranged between the liquid management structure 310 and the bottom core wrap sheet 320. The liquid management structure 310 comprises a first connecting portion which is attached to the bottom core wrap sheet 320 forming a first attachment zone 313a. The liquid management structure 310 comprises a second connecting portion which is attached to the bottom core wrap sheet 320 forming a second attachment zone 313b. The first attachment zone 313a and the second attachment zone 313b diverge from the crotch region in the direction of a front and rear transverse edge of absorbent article. The center line of the first attachment zone 313a and the second attachment zone 313b may have a curved shape. Seen in a top view of the absorbent core 300, the liquid management structure 310 has a total surface area of S1 and the absorbent core has a surface area of S0. S1 and S0 are substantially the same.

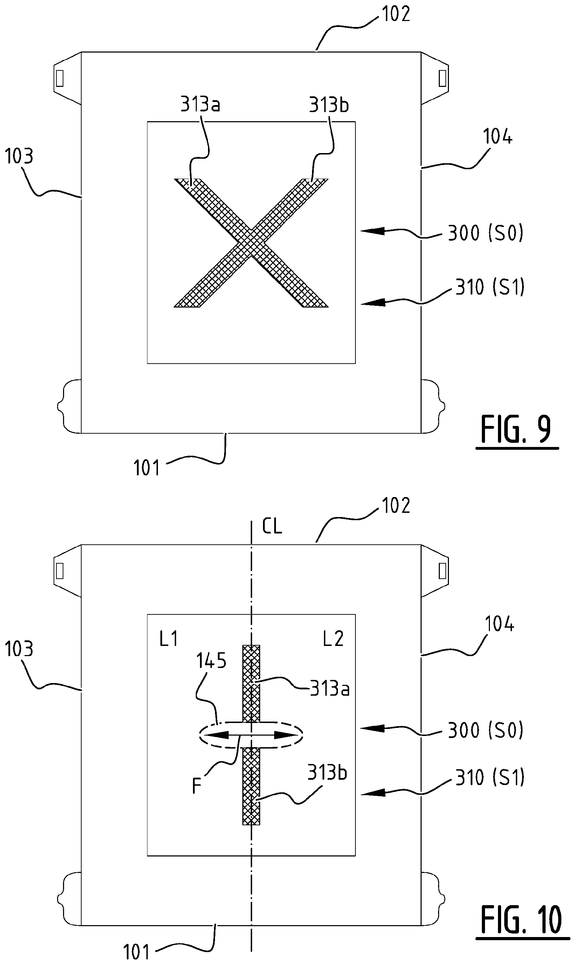

In the embodiment of Figure 9, the absorbent core 300 comprises a liquid management structure 310, a bottom core wrap sheet 320 (not shown), and absorbent material 330 arranged between the liquid management structure 310 and the bottom core wrap sheet 320. The liquid management structure 310 comprises a first connecting portion which is attached to the bottom core wrap sheet 320 forming a first attachment zone 313a. The liquid management structure 310 comprises a second connecting portion which is attached to the bottom core wrap sheet 320 forming a second attachment zone 313b. The first attachment zone 313a and the second attachment zone 313b together form a substantially X-shaped zone. Preferably the crossing point is on a longitudinal center line of the absorbent core 300 extending between the transverse edges. Preferably the crossing point is in the crotch portion of the absorbent core 300. Seen in a top view of the absorbent core 300, the liquid management structure 310 has a total surface area of S1 and the absorbent core has a surface area of S0. S1 and S0 are substantially the same.

In the embodiment of Figure 10, the absorbent core 300 comprises a liquid management structure 310, a bottom core wrap sheet 320 (not shown), and absorbent material 330 arranged between the liquid management structure 310 and the bottom core wrap sheet 320. The liquid management structure 310 comprises a first connecting portion which is attached to the bottom core wrap sheet 320 forming a first attachment zone 313a. The liquid management structure 310 comprises a second connecting portion which is attached to the bottom core wrap sheet 320 forming a second attachment zone 313b. Between the first attachment zone 313a and the second attachment zone 313b a bridge zone 145 is formed which allows a liquid flow F between the first and the second longitudinal edge of the absorbent core 300 by capillary action through the absorbent material and/or by mass flow, such that upon wetting of the absorbent material, a first channel and a second channel in front of one another are created, wherein the bridge zone 145 extends between the first and second channel. A minimum distance between said first and second channel is preferably larger than 3 mm and more preferably larger than 5 mm. Seen in a top view of the absorbent core 300, the liquid management structure 310 has a total surface area of S1 and the absorbent core has a surface area of S0. S1 and S0 are substantially the same.

In the embodiments of Figures 8, 9 and 10 only one liquid management structure 310 is provided which comprises two attachment zones 313a, 313b and which comprises no free edges. In other words, seen from the top direction of the absorbent core, the liquid management structure has a total surface area of S1 which is substantially equal to S0 which is the surface area of the absorbent core defined by an area covered by the absorbent material plus an area of the two attachment zones 313a, 313b. It is however clear to the skilled person that a similar pattern of attachment zones 313a, 313b may be provided by 1 or 2 separate liquid managing structures, wherein each liquid managing structure may haves none, 1, 2, 3 or 4 free edges, wherein the total combined surface area S1 of the liquid managing structure(s) is smaller than 90% of S0 (see Figures 6A-6D).

Whilst the principles of the invention have been set out above in connection with specific embodiments, it is to be understood that this description is merely made by way of example and not as a limitation of the scope of protection which is determined by the appended claims.

## Claims

1. An absorbent article comprising a liquid pervious topsheet (100), a liquid impervious backsheet (200), and an absorbent core (300) positioned between the liquid pervious topsheet and the liquid impervious backsheet; said absorbent article having a first and second longitudinal edge (103, 104) and a first and second transverse edge (101, 102); wherein the absorbent core comprises a bottom core wrap sheet (320), absorbent material (330), and a liquid management structure (310), wherein the liquid management structure (310) covers at least a surface portion of the absorbent material and comprises at least one connecting portion (311) which is attached to the bottom core wrap sheet forming at least one attachment zone (313), **characterized in that** the liquid management structure has a grammage of at least 19 g/m² and comprises meltblown nonwoven material and/or spunbond nonwoven material with a grammage of at least 15 g/m², **in that** the absorbent material (330) is positioned directly between the bottom core wrap sheet (320) and the liquid management structure (310), and **in that** the liquid management structure (310) is positioned directly between the absorbent material and the liquid pervious topsheet.

2. The absorbent article according to any one of the preceding claims, wherein the liquid management structure has a grammage of at least 20 g/m².

3. The absorbent article according to any one of the preceding claims, wherein the liquid management structure is in contact with an upper surface portion of the absorbent material (330).

4. The absorbent article according to any one of the preceding claims, wherein the liquid management structure (310) extends over substantially an entire upper surface of the absorbent material (330).

5. The absorbent article according to any one of the preceding claims, wherein seen from the top direction of the absorbent core, the liquid management structure has a total surface area of S1, the absorbent core has a surface area of S0 defined by an area covered by the absorbent material plus an area of the at least one attachment zone, wherein S1 is smaller than 90% of S0.

6. The absorbent article according to claim 5, wherein S1 is smaller than 80% of S0, preferably S1 is smaller than 70% of S0, more preferably S1 is smaller than 60% of S0, even more preferably S1 is smaller than 50% of S0, most preferably S1 is smaller than 40% of S0.

7. The absorbent article according to any one of the preceding claims, wherein the at least one attachment zone (313) is formed by heat-sealing the at least one portion of the liquid management structure (310) to the bottom core wrap sheet (320).

8. The absorbent article according to any one of the preceding claims, wherein the liquid management structure comprises a liquid acquisition layer; and/or a liquid distribution layer.

9. The absorbent article according to any one of the preceding claims, wherein in the at least one attachment zone (313) substantially no absorbent material is present between the liquid management structure (310) and the bottom core wrap sheet (320).

10. The absorbent article according to any one of the preceding claims, wherein the at least one attachment zone (313) extends from a crotch region in the direction of the first and/or second transverse edge of the absorbent core, and/or the at least one attachment zone (313) extends in the direction from the first longitudinal edge to the second longitudinal edge of the absorbent core.

11. The absorbent article according to any one of the preceding claims, wherein the absorbent core is provided with at least a second attachment zone, said second attachment zone extending in the transversal direction of the absorbent core in between the first and second longitudinal edge.

12. The absorbent article according to any one of the preceding claims, wherein the at least one attachment zone formed by the liquid management structure (310) and the bottom core wrap (320) comprises a permanent attachment, which is configured to remain attached when the absorbent material swells upon wetting; and/or wherein the at least one attachment zone formed by the liquid management structure (310) and the bottom core wrap (320) comprises a semi-permanent attachment, which is configured to release when the absorbent material swells upon wetting.

13. The absorbent article according to any one of the preceding claims, wherein a length of the at least one attachment zone is larger than 10% of the length of the absorbent core, more preferably larger than 30%, even more preferably larger than 50%.

14. The absorbent article according to any one of the preceding claims, wherein the at least one attachment zone comprises a first attachment zone and a second attachment zone, wherein said first and second attachment zones extend next to each other from the crotch region in the direction of the first and/or the second transverse edge.

15. The absorbent article according to any one of the preceding claims, wherein the at least one attachment zone is a continuous zone.

## Patentansprüche

1. Saugfähiger Artikel, der eine flüssigkeitsdurchlässige Decklage (100), eine flüssigkeitsundurchlässige rückseitige Lage (200) und einen absorbierenden Kern (300) aufweist, der zwischen der flüssigkeitsdurchlässigen Decklage und der flüssigkeitsundurchlässigen rückseitigen Lage angeordnet ist, wobei der saugfähige Artikel eine erste und zweite Längskante (103, 104) und eine erste und zweite Querkante (101, 102) aufweist; wobei der absorbierende Kern eine untere Kernumhüllungslage (320), Absorptionsmaterial (330) und eine Flüssigkeitshandhabungsstruktur (310) aufweist, wobei die Flüssigkeitshandhabungsstruktur (310) wenigstens einen Oberflächenbereich des Absorptionsmaterials abdeckt und wenigstens einen Verbindungsbereich (311) aufweist, der an der unteren Kernumhüllungslage angebracht ist, die wenigstens eine Anbringungszone (313) bildet,
**dadurch gekennzeichnet, dass**
die Flüssigkeitshandhabungsstruktur eine Grammatur von wenigstens 19 g/m² und schmelzgeblasenes Vliesmaterial und/oder Spinnvlies-Vliesmaterial mit einer Grammatur von wenigstens 15 g/m² aufweist,
darin dass das Absorptionsmaterial (330) direkt zwischen der unteren Kernumhüllungslage (320) und der Flüssigkeitshandhabungsstruktur (310) angeordnet ist, und
darin, dass die Flüssigkeitshandhabungsstruktur (310) direkt zwischen dem Absorptionsmaterial und der flüssigkeitsdurchlässigen Decklage angeordnet ist.

2. Saugfähiger Artikel nach einem der vorhergehenden Ansprüche, wobei die Flüssigkeitshandhabungsstruktur eine Grammatur von wenigstens 20 g/m² hat.

3. Saugfähiger Artikel nach einem der vorhergehenden Ansprüche, wobei die Flüssigkeitshandhabungsstruktur in Kontakt mit einem Oberflächenbereich des Absorptionsmaterials (330) steht.

4. Saugfähiger Artikel nach einem der vorhergehenden Ansprüche, wobei die Flüssigkeitshandhabungsstruktur (310) über im Wesentlichen eine gesamte Oberfläche des Absorptionsmaterials (330) verläuft.

5. Saugfähiger Artikel nach einem der vorhergehenden Ansprüche, wobei von einer oberen Richtung des Absorptionskerns betrachtet, die Flüssigkeitshandhabungsstruktur eine Gesamtfläche S1 hat, der Absorptionskern eine Oberfläche S0 hat, die durch eine Fläche definiert ist, die vom Absorptionsmaterial plus einer Fläche von der wenigstens einen Anbringungszone bedeckt ist, wobei S1 kleiner als 90 % von S0 ist.

6. Saugfähiger Artikel nach Anspruch 5, wobei S1 kleiner als 80 % von S0, S1 vorzugsweise kleiner als 70 % von S0 ist, S1 bevorzugter kleiner als 60 % von S0 ist, S1 besonders bevorzugt kleiner als 50 % von S0 ist, S1 am meisten bevorzugt kleiner als 40 % von S0 ist.

7. Saugfähiger Artikel nach einem der vorhergehenden Ansprüche, wobei die wenigstens eine Anbringungszone (313) durch eine Heißversiegelung von dem wenigstens einem Bereich der Flüssigkeitshandhabungsstruktur (310) an der unteren Kernumhüllungslage (320) ausgebildet ist.

8. Saugfähiger Artikel nach einem der vorhergehenden Ansprüche, wobei die Flüssigkeitshandhabungsstruktur eine Flüssigkeitsaufnahmeschicht und/oder eine Flüssigkeitsverteilungsschicht aufweist.

9. Saugfähiger Artikel nach einem der vorhergehenden Ansprüche, wobei in der wenigstens einen Anbringungszone (313) im Wesentlichen kein Absorptionsmaterial zwischen der Flüssigkeitshandhabungsstruktur (310) und der unteren Kernumhüllungslage (320) vorhanden ist.

10. Saugfähiger Artikel nach einem der vorhergehenden Ansprüche, wobei die wenigstens eine Anbringungszone (313) von einem Schrittbereich in der Richtung der ersten und/oder zweiten Querkante des Absorptionskerns verläuft und/oder die wenigstens eine Anbringungszone (313) in der Richtung von der ersten Längskante zur zweiten Längskante des Absorptionskerns verläuft.

11. Saugfähiger Artikel nach einem der vorhergehenden Ansprüche, wobei der Absorptionskern mit wenigstens einer zweiten Anbringungszone vorgesehen ist, wobei die zweite Anbringungszone in der Querrichtung des Absorptionskerns zwischen der ersten und der zweiten Längskante verläuft.

12. Saugfähiger Artikel nach einem der vorhergehenden Ansprüche, wobei die wenigstens eine Anbringungszone, die durch die Flüssigkeitshandhabungsstruktur (307) und die untere Kernumhüllung (320) ausgebildet ist, eine dauerhafte Anbringung aufweist, die ausgestaltet ist, angebracht zu bleiben, wenn das Absorptionsmaterial bei der Benässung anschwillt; und/oder wobei die wenigstens eine Anbringungszone, die durch die Flüssigkeitshandhabungsstruktur (310) ausgebildet ist, und die untere Kernumhüllung (320) eine halb- dauerhafte Anbringung aufweist, die ausgestaltet ist, sich zu lösen, wenn das Absorptionsmaterial bei der Benässung anschwillt.

13. Saugfähiger Artikel nach einem der vorhergehenden Ansprüche, wobei eine Länge der wenigstens einen Anbringungszone größer als 10 % der Länge des Absorptionskerns, bevorzugter größer als 30 %, noch bevorzugter größer als 50 % ist.

14. Saugfähiger Artikel nach einem der vorhergehenden Ansprüche, wobei die wenigstens eine Anbringungszone eine erste Anbringungszone und eine zweite Anbringungszone aufweist, wobei die ersten und zweiten Anbringungszone nebeneinander vom Schrittbereich in Richtung der ersten und/oder der zweiten Querkante verlaufen.

15. Saugfähiger Artikel nach einem der vorhergehenden Ansprüche, wobei die wenigstens eine Anbringungszone eine durchgängige Zone ist.

## Revendications

1. Article absorbant comprenant une feuille supérieure perméable au liquide (100), une feuille arrière imperméable au liquide (200) et un noyau absorbant (300) positionné entre la feuille supérieure perméable au liquide et la feuille arrière imperméable au liquide ; ledit article absorbant présentant un premier et un second bord longitudinal (103, 104) et un premier et un second bord transversal (101, 102) ; dans lequel le noyau absorbant comprend une feuille d'enveloppe de noyau inférieure (320), un matériau absorbant (330) et une structure de rétention de liquide (310),
dans lequel la structure de rétention de liquide (310) recouvre au moins une partie de surface du matériau absorbant et comprend au moins une partie de liaison (311) qui est fixée sur la feuille d'enveloppe de noyau inférieure formant au moins une zone de fixation (313), **caractérisé en ce que** la structure de rétention de liquide présente un grammage supérieur ou égal à 19 g/m2 et comprend un matériau non tissé, soufflé à l'état fondu et/ou un matériau non tissé filé-lié présentant un grammage supérieur ou égal à 15 g/m2, **en ce que** le matériau absorbant (330) est positionné directement entre la feuille d'enveloppe de noyau inférieure (320) et la structure de rétention de liquide (310), et **en ce que** la structure de rétention de liquide (310) est positionnée directement entre le matériau absorbant et la feuille supérieure perméable au liquide.

2. Article absorbant selon l'une quelconque des revendications précédentes, dans lequel la structure de rétention de liquide présente un grammage supérieur ou égal à 20 g/m2.

3. Article absorbant selon l'une quelconque des revendications précédentes, dans lequel la structure de rétention de liquide est en contact avec une partie de surface supérieure du matériau absorbant (330).

4. Article absorbant selon l'une quelconque des revendications précédentes, dans lequel la structure de rétention de liquide (310) s'étend sensiblement sur la totalité de la surface supérieure du matériau absorbant (330).

5. Article absorbant selon l'une quelconque des revendications précédentes, dans lequel, en vue de dessus du noyau absorbant, la structure de rétention de liquide présente une superficie totale égale à S1, le noyau absorbant présente une superficie égale à S0 définie par une surface recouverte par le matériau absorbant plus une surface de la au moins une zone de fixation, dans lequel S1 est inférieur à 90% de S0.

6. Article absorbant selon la revendication 5, dans lequel S1 est inférieur à 80% de S0, de préférence, S1 est inférieur à 70% de S0, plus préférablement, S1 est inférieur à 60% de S0, encore plus préférablement, S1 est inférieur à 50% de S0, le plus préférablement, S1 est inférieur à 40% de S0.

7. Article absorbant selon l'une quelconque des revendications précédentes, dans lequel la au moins une zone de fixation (313) est formée par thermocollage de la au moins une partie de la structure de rétention de liquide (310) sur la feuille d'enveloppe de noyau inférieure (320).

8. Article absorbant selon l'une quelconque des revendications précédentes, dans lequel la structure de rétention de liquide comprend une couche de réception de liquide ; et/ou une couche de répartition de liquide.

9. Article absorbant selon l'une quelconque des revendications précédentes, dans lequel, dans la au moins une zone de fixation (313), sensiblement aucun matériau absorbant n'est présent entre la structure de rétention de liquide (310) et la feuille d'enveloppe de noyau inférieure (320).

10. Article absorbant selon l'une quelconque des revendications précédentes, dans lequel la au moins une zone de fixation (313) s'étend à partir d'une section d'entrejambe dans la direction du premier et/ou second bord transversal du noyau absorbant, et/ou la au moins une zone de fixation (313) s'étend dans la direction à partir du premier bord longitudinal vers le second bord longitudinal du noyau absorbant.

11. Article absorbant selon l'une quelconque des revendications précédentes, dans lequel le noyau absorbant comporte au moins une seconde zone de fixation, ladite seconde zone de fixation s'étendant dans la direction transversale du noyau absorbant entre le premier et le second bord longitudinal.

12. Article absorbant selon l'une quelconque des revendications précédentes, dans lequel la au moins une zone de fixation formée par la structure de rétention de liquide (310) et l'enveloppe de noyau inférieure (320) comprend un élément de fixation permanent, qui est configuré de manière à rester fixé lorsque le matériau absorbant gonfle en étant mouillé ; et/ou dans lequel la au moins une zone de fixation formée par la structure de rétention de liquide (310) et l'enveloppe de noyau inférieure (320) comporte un élément de fixation semi-permanent, qui est configuré de manière à se libérer lorsque le matériau absorbant gonfle en étant mouillé.

13. Article absorbant selon l'une quelconque des revendications précédentes, dans lequel une longueur de la au moins une zone de fixation est supérieure à 10% de la longueur du noyau absorbant, plus préférablement supérieure à 30%, encore plus préférablement, supérieure à 50%.

14. Article absorbant selon l'une quelconque des revendications précédentes, dans lequel la au moins une zone de fixation comprend une première zone de fixation et une seconde zone de fixation, dans lequel lesdites première et seconde zones de fixation s'étendent à proximité l'une de l'autre à partir de la section d'entrejambe en direction du premier et/ou du second bord transversal.

15. Article absorbant selon l'une quelconque des revendications précédentes, dans lequel la au moins une zone de fixation est une zone continue.
